Europäisches Patentamt

European Patent Office

Office européen des brevets

(19)

(11) Numéro de publication: **0 272 945 B1**

# FASCICULE DE BREVET EUROPEEN

(12)

(45) Date de publication de fascicule du brevet:
24.07.91

(51) Int. Cl.⁵: **C07C 53/42**, C07C 51/58

(21) Numéro de dépôt: 87402375.7

(22) Date de dépôt: **22.10.87**

(54) Procédé catalytique de carbonylation des alcanes.

(30) Priorité: **20.11.86 FR 8616143**
**19.02.87 FR 8702140**
**24.07.87 FR 8710567**

(43) Date de publication de la demande:
**29.06.88 Bulletin 88/26**

(45) Mention de la délivrance du brevet:
**24.07.91 Bulletin 91/30**

(84) Etats contractants désignés:
**AT BE CH DE ES FR GB GR IT LI LU NL SE**

(56) Documents cités:
**FR-A- 1 490 433**
**US-A- 3 367 953**

(73) Titulaire: **ATOCHEM**
**4 & 8, Cours Michelet La Défense 10**
**F-92800 Puteaux(FR)**

(72) Inventeur: **Delavarenne, Serge**
**35 Rue de la Croix de Fer**
**F-78100 Saint Germain En Laye(FR)**
Inventeur: **Fauconet, Michel**
**63 Rue Hirschauer**
**F-57500 St. Avold(FR)**
Inventeur: **Simon, Michel**
**35 Rue de Stalingrad**
**F-62440 Harnes(FR)**
Inventeur: **Sommer, Jean**
**8 Rue de Stockholm**
**F-67000 Strasbourg(FR)**

(74) Mandataire: **Rochet, Michel et al**
**ATOCHEM Département Propriété Industriel-**
**le La Défense 10 Cédex 42**
**F-92091 Paris La Défense(FR)**

## Description

On connaît déjà un procédé de préparation de fluorure d'isobutyryle à partir d'un mélange anhydre de propylène, monoxyde de carbone et fluorure d'hydrogène. En particulier le brevet US-A-4.499.029 consiste à faire passer un tel mélange à travers au moins deux zones réactionnelles disposées en série et à ajouter au mélange réactionnel, entre les zones de réaction, des quantités progressives de propylène et de monoxyde de carbone anhydres, ledit procédé étant effectué avec un temps de séjour dans les zones réactionnelles de 15 secondes à 10 minutes, sous une pression de 1 à 150 bars, à une température de 0° à 100°C, le rapport molaire $C_3H_6$/CO/HF dans le mélange réactionnel étant compris entre 1/5/5 et 1/30/200. Toutefois le prix de revient du fluorure d'isobutyryle ainsi obtenu est assez élevé, étant donné que la matière première de ce procédé, à savoir le propylène, est elle-même obtenue par déshydrogénation des alcanes des coupes pétrolières ou par vapocraquage d'hydrocarbures.

Il est déjà connu par H.HOGEVEEN et C.F.ROOBEEK, Rec. Trav. Chim. Pays-Bas, 91 (1972) pages 137-140, de faire réagir à 0°C un mélange équimolaire de n-butane et de monoxyde de carbone en présence de pentafluorure d'antimoine $SbF_5$ en solution dans $SO_2ClF$. Cette réaction conduit à la formation d'un mélange d'ions sec-butyloxocarbonium (74%), tert-butylcarbonium (25%) et tert-butyloxocarbonium (1%). Par le même document, il est connu de carbonyler le propane à 0°C, dans un solvant ($SO_2ClF$) et en présence de pentafluorure d'antimoine, le rapport molaire $C_3H_8$/CO étant compris entre 1 et 9. Il est également connu par N. YONEDA et al., Chemical Letters (Chemical Society of Japan), (1983), pages 17-18 de carbonyler des alcanes ramifiés comportant au moins 5 atomes de carbone, à la température de 30°C, en présence du superacide HF-$SbF_5$ (rapport molaire HF/$SbF_5$ égal à 5), le rapport molaire alcane/HF étant égal à 0,1. Par ailleurs il est connu par G. OLAH et al.,Journal of the American Chemical Society, 95, pages 4939 et suiv., que :
- à une température comprise entre -10°C et -103°C, dans un solvant ($SO_2ClF$) et en présence du superacide $HSO_3F$-$SbF_5$, s'établit un équilibre entre le propane et le cation isopropyle, et
- à la température de -78°C, dans un solvant ($SO_2ClF$) et en présence d'un système superacide comprenant du fluorure d'hydrogène et du pentafluorure d'antimoine, s'établit un équilibre entre le méthyl-2 propane (ou isobutane) et l'ion triméthylcarbénium.

E. HOGEVEEN a déjà décrit dans Adv.Phys.org.Chem.,10,32 (1973) la réaction de décarbonylation du cation pivaloyle à -70°C, soit dans un mélange équimolaire de fluorure d'hydrogène et de pentafluorure d'antimoine soit dans un mélange de 2 parties en volume de $SO_2ClF$ pour 1 partie en volume de pentafluorure d'antimoine, pour former le cation tertio-butyle.

On remarquera qu'en général ces documents antérieurs s'intéressent exclusivement aux cinétiques de protonation des alcanes ou de décarbonylation à très basse température et ne décrivent pas d'espèces covalentes susceptibles d'être obtenues par la mise en jeu de ces réactions. En particulier aucun d'entr'eux ne décrit l'obtention de fluorures d'acide. D'autre part aucun d'entr'eux n'a montré la possibilité de régénérer le superacide utilisé pour la protonation.

D'autre part le brevet US-A-4.582.571 mentionne la possibilité de former du fluorure d'isobutyryle par réaction de monoxyde de carbone, de propane, de fluorure d'hydrogène anhydre et de pentafluorure d'antimoine sous une pression supérieure à 100 bars et à une température proche de 100 C. Toutefois d'une part ce document est muet sur les proportions respectives des différents réactifs et d'autre part prévoit la mise en jeu de conditions de pression et de température trop sévères d'un point de vue industriel. Par ailleurs la demanderesse a découvert que la nature du fluorure d'acide formé par un tel procédé dépend de manière surprenante de la valeur choisie pour le rapport molaire CO/propane.

Le problème que la présente invention s'attache à résoudre consiste en la fabrication d'un fluorure d'acide à partir d'un alcane (les fluorures de propionyle et d'isobutyryle à partir du propane, le fluorure de pivaloyle à partir de l'isobutane) par un procédé ne présentant ni l'inconvénient (mentionné précédemment) du prix de revient élevé de la matière première puisque l'alcane est extrait directement des coupes pétrolières ni l'autre inconvénient des conditions de température trop sévères du brevet US-A 4.582.571. Dans un premier temps la demanderesse s'est livrée à l'étude de la carbonylation d'alcanes possédant 3 à 4 atomes de carbone en présence de différents systèmes superacides afin de déterminer un système susceptible de conduire, de manière efficace et économique, à l'obtention de fluorure d'acide. Dans un second temps la demanderesse s'est livrée à l'étude de la séparation du composé obtenu afin de déterminer le moyen susceptible d'assurer, de la manière la plus efficace possible, la régénération du superacide choisi.

Le procédé selon l'invention se définit donc comme un procédé catalytique de fabrication de fluorure d'acide à partir de monoxyde de carbone, de fluorure d'hydrogène et d'un flux d'hydrocarbures aliphatiques comprenant à titre principal au moins un alcane possédant 3 à 4 atomes de carbone caractérisé par le fait

2

qu'il comprend la succession d'étapes suivante:

(a) introduction d'au moins un fluide choisi parmi le monoxyde de carbone et ledit flux d'hydrocarbures dans un réacteur en présence d'un système catalytique superacide constitué essentiellement de fluorure d'hydrogène et de pentafluorure d'antimoine SbF5.

(b) le cas échéant, s'il n'a pas déjà été introduit au cours de l'étape (a), introduction dans le réacteur d'un fluide choisi parmi le monoxyde de carbone et ledit flux d'hydrocarbures, le réacteur étant soumis à une température au plus égale à 60°C de manière à former, à titre principal un complexe constitué du cation alkyloxocarbonium et de l'anion SbF$_6^-$ ,

(c) conversion dudit complexe en fluorure d'acide,

(d) séparation du fluorure d'acide,

(e) récupération du système catalytique superacide, et

(f) le cas échéant, ajustement de la quantité de fluorure d'hydrogène à la constitution du système catalytique superacide mis en oeuvre dans l'étape (a).

Ainsi le procédé selon l'invention comprend obligatoirement les quatre étapes désignées (a), (c), (d) et (e). Dans ce cas le monoxyde de carbone et le flux comprenant l'alcane à titre principal sont introduits simultanément dans le réacteur. Alternativement il peut n'être pas nécessaire de mettre simultanément le monoxyde de carbone et le flux comprenant l'alcane à titre principal en présence du système catalytique superacide. Dans ce second cas le monoxyde de carbone et ledit flux d'hydrocarbures seront introduits séparément dans le réacteur grâce à l'étape supplémentaire (b).

Pour la bonne compréhension de l'invention il convient de préciser que :

- par "alcane à titre principal" on entend que le flux d'hydrocarbures aliphatiques peut comprendre, à côté de l'alcane (propane, n-butane ou isobutane), de faibles proportions d'alcènes ou alcynes ayant un faible nombre d'atomes de carbone, tels que notamment butène, propyne ou propylène.

- par "cation alkyloxocarbonium à titre principal" on entend que la réaction engendrée par le système catalytique superacide selon l'invention conduit essentiellement à la formation de ce cation, à côté de proportions mineures de carbocations soit dérivés des autres hydrocarbures aliphatiques éventuellement présents dans le milieu réactionnel soit provenant de réarrangements desdits carbocations ou du cation alkyloxocarbonium. Ainsi le cation isopropyloxocarbonium (également dénommé isobutyryle) peut, dans certaines conditions, etre formé à partir du propane, le cation éthyloxocarbonium (encore dénommé propionyle) est formé à partir du propane, le cation ter-butyloxocarbonium (également dénommé pivaloyle) est formé à partir de l'isobutane.

Par ailleurs le contenu de la description qui va suivre a pour objet de préciser le mode de réalisation de chacune des étapes du procédé selon la présente invention. L'étape (a) a pour objet de mettre soit le monoxyde de carbone, soit le flux comprenant l'alcane à titre principal, soit leur mélange, en contact avec le système catalytique superacide selon l'invention. Cette mise en présence s'effectue dans un réacteur qui peut être de type autoclave ou bien de type tubulaire ou de tout autre type convenant à la mise en contact des réactifs. D'autre part au moment de l'introduction dudit (desdits) réactifs dans le réacteur, celui-ci contient déjà le système catalytique comprenant essentiellement le fluorure d'hydrogène et le pentafluorure d'antimoine SbF5 en proportions telles qu'ils constituent une phase homogène.

Lors de l'étape (b), facultative comme indiqué précédemment, l'autre composant est introduit dans le réacteur. Ce n'est qu'à partir de ce moment que, monoxyde de carbone et alcane étant en contact avec le système catalytique superacide, un complexe constitué du cation alkyloxocarbonium et de l'anion SbF$_6^-$ va commencer à se former à titre principal. La présence dudit cation est confirmée par prélèvement du milieu réactionnel à ce stade et analyse, notamment par résonance magnétique nucléaire du proton. Par cette analyse on obtient un spectre comprenant :

- dans le cas du cation propionyle : un triplet à 2,2 ppm environ (3H) et un quadruplet à 4,3 ppm environ (2H).

- dans le cas du cation isobutyryle : un doublet à 2,1 ppm environ (6H) et un heptuplet à 4,4 ppm environ (1H).

- dans le cas du cation pivaloyle : un singulet à 2,0 ppm environ (9H).

Pour que la formation dudit complexe s'effectue le plus efficacement possible, il est souhaitable que les conditions opératoires dans le réacteur soient choisies comme suit :

- un rapport molaire CO/alcane au moins égal à 0,1 et de préférence compris entre 0,1 et 30 environ ; lorsque l'alcane est le propane, un rapport molaire CO/propane supérieur à 7:3 permet la formation de fluorure de propionyle à titre principal tandis qu'un rapport molaire non supérieur à 7:3 permet la formation de fluorure d'isobutyryle à titre principal.

- un rapport molaire HF/SbF5 compris entre 1 et 30 environ,

- une température comprise entre -80°C et +60°C environ,

3

EP 0 272 945 B1

Le complexe formé à l'issue de l'étape (b) est utilisé, notamment grâce aux modes de réalisation décrits ci-après, pour fabriquer avec un bon rendement le fluorure d'acide.

Selon un premier mode de réalisation du procédé selon l'invention, on convertit le complexe en fluorure d'acide par intervention d'au moins un moyen pour déplacer l'équilibre entre ledit complexe et le fluorure d'acide vers la formation de ce dernier. Parmi les moyens d'intervention, dans le cadre de l'étape (c), pour déplacer cet équilibre chimique, on peut citer notamment l'addition d'une espèce chimique capable de diminuer significativement l'acidité du milieu réactionnel. Parmi de telles espèces, on choisira de préférence le fluorure d'hydrogène. Des moyens d'intervention de nature physique, par exemple thermique, peuvent également être envisagés dans le cadre de l'étape (c).

L'équilibre chimique précité ayant été suffisamment déplacé, il convient alors dans l'étape (d) de séparer une proportion substantielle du fluorure d'acide des autres constituants du milieu réactionnel. Connaissant les températures d'ébullition à la pression atmosphérique du fluorure d'hydrogène (20°C), du pentafluorure d'antimoine (150°C) et du fluorure d'acide (60°C dans le cas du fluorure d'isobutyryle par exemple), ainsi que les quantités respectives des différents composants en présence, l'homme de l'art est en mesure de choisir la méthode de séparation la plus appropriée.

Toujours dans le cadre de ce premier mode de réalisation, le procédé selon la présente invention prévoit en outre que le système catalytique superacide et éventuellement le fluorure d'acide résiduel soient récupérés au cours de l'étape (e). Lorsque le procédé est effectué de manière discontinue, le système catalytique superacide, seul ou en mélange avec du fluorure d'hydrogène et éventuellement une partie du fluorure d'acide n'ayant pas été séparé, est récupéré pour être réutilisé pour la réaction suivante. Lorsque le procédé est effectué de manière continue le système catalytique superacide, le cas échéant en mélange avec du fluorure d'hydrogène et/ou du fluorure d'acide, est recyclé vers le réacteur. Lorsque du fluorure d'hydrogène a été ajouté au cours de l'étape (c), le recyclage peut être effectué après élimination partielle du fluorure d'hydrogène. Dans ce cas un mode de réalisation particulier consiste à ajuster, grâce à l'étape (f), la quantité de fluorure d'hydrogène à la constitution du système catalytique superacide mis en oeuvre dans l'étape (a). Le fluorure d'hydrogène éliminé à ce stade peut à son tour être recyclé, par exemple vers le point d'intervention de l'étape (c) où il permettra au moins partiellement de constituer l'appoint nécessaire au déplacement de l'équilibre chimique sus-mentionné.

La réaction du procédé selon la présente invention peut être commodément effectuée sous pression atmosphérique. Pour des raisons de cinétique et d'économie d'exploitation industrielle, il pourra également être avantageux de procéder sous une pression supérieure à la pression atmosphérique, par exemple sous une pression allant jusqu'à 250 bars environ et de préférence ne dépassant pas 40 bars environ. L'homme de l'art est capable de choisir, en fonction du niveau de la pression, d'une part le temps de séjour du milieu réactionnel dans le réacteur et d'autre part la température appropriée. Le temps de séjour dans le réacteur est généralement compris entre 0,1 et 300 minutes. De plus dans le cas où la pression de réaction est notablement supérieure à la pression atmosphérique, il peut être souhaitable d'abaisser la pression à l'issue de l'étape (c) et de recomprimer les fluides récupérés notamment au cours l'étape (e) et, le cas échéant, au cours de l'étape (f) jusqu'à la pression du réacteur.

Le procédé selon l'invention permet d'obtenir avec une cinétique et un rendement satisfaisants, après au moins une étape de purification bien connue de l'homme de l'art telle que la distillation fractionnée, du fluorure d'acide ayant un degré de pureté adapté pour les utilisations ultérieures. En effet le fluorure d'acide consitue un intermédiaire de synthèse permettant notamment la production de l'acide carboxylique correspondant par hydrolyse. Par exemple le fluorure d'isobutyryle constitue un intermédiaire de synthèse particulièrement important permettant notamment la production d'acide méthacrylique successivement par hydrolyse en acide isobutyrique puis oxydéshydrogénation de ce dernier ou bien la production de méthacrylate de méthyle par méthanolyse en isobutyrate de méthyle puis oxydéshydrogénation de ce dernier. Conformément à l'objectif de la présente invention, ces composés sont obtenus avec un prix de revient modéré en raison du choix d'un alcane comme matière première de départ.

Les exemples ci-après ont pour objet d'illustrer la présente invention, sans en limiter la portée en aucune manière.

EXEMPLES 1 à 4

Dans un réacteur en polymonochlorotrifluoroéthylène de volume 0,1 litre on introduit à 20°C, sous agitation et sous atmosphère d'azote, du pentafluorure d'antimoine et du fluorure d'hydrogène dans le rapport molaire $HF/SbF_5$ égal à 4. Ce mélange est ensuite transféré dans un réacteur autoclave en acier inoxydable de volume 0,3 litre. La température T du mélange (exprimée en degrés Celsius) est ensuite amenée à la valeur indiquée dans le tableau I puis le mélange de propane et de monoxyde de carbone

4

dans le rapport molaire CO/C$_3$H$_8$ indiqué dans le tableau I est introduit par une turbine jusqu'à atteindre la pression P mentionnée dans le tableau I (exprimée en bars). Deux régimes opératoires peuvent être alors appliqués, à savoir :

- un régime "statique" (noté s) : dans ce cas la totalité des réactifs est introduite au début et la pression indiquée dans le tableau I est la pression initiale, ou bien
- un régime "dynamique" (noté d) : dans ce cas l'addition de propane et de monoxyde de carbone se poursuit durant toute la réaction avec un temps de contact du gaz avec la phase liquide d'environ 9 secondes, la pression indiquée dans le tableau étant constante durant l'essai.

Dans chaque cas la durée de l'essai est de 1 heure.A la fin de l'essai (régime "statique") ou au cours de l'essai (régime "dynamique"), la phase gazeuse est détendue à une température ne dépassant pas 25° C et analysée en ligne dans une série de chromatographes permettant d'identifier et doser :

- d'une part le propane et le monoxyde de carbone n'ayant pas réagi, de manière à calculer le taux de conversion T.C. (exprimé en % et indiqué au tableau I ci-après), égal au nombre de moles de propane consommées sur le nombre de moles de propane introduites.
- d'autre part les produits secondaires gazeux issus de la réaction, constituant généralement un mélange d'hydrogène, méthane et éthane dont les proportions molaires dans ledit mélange sont celles indiquées au tableau I ci-après.

Par ailleurs la phase liquide contenue dans le réacteur est soumise à une hydrolyse totale à 0° C, puis la solution aqueuse obtenue est analysée par chromatographie en phase gazeuse de manière à identifier et doser les composés organiques formés par hydrolyse des produits de la réaction. Ceux-ci constituent généralement un mélange d'acide isobutyrique (AIB), d'acide propionique (AP) et de composés divers (globalement notés "autres" dans le tableau I ci-après), ceux-ci comprenant notamment l'acide acétique, l'acide n-butyrique, le méthanol et l'acétone. Les proportions molaires des acides AIB et AP dans le mélange sont indiqués au tableau I ci-après.

EXEMPLES 5 à 9

Dans un réacteur en polymonochlorotrifluoroéthylène de volume 3 ml muni de deux robinets et contenant un mélange de fluorure d'hydrogène et de pentafluorure d'antimoine dans un rapport molaire HF/SbF$_5$ égal à 3,8 plonge un tube en polymonochlorotrifluoroéthylène par lequel on introduit, selon un débit de 210 ml/heure, un mélange de monoxyde de carbone et de propane dans le rapport molaire CO/C$_3$H$_8$ indiqué au tableau ci-après (l'exemple 5 est comparatif). Ce réacteur est maintenu sous pression atmosphérique et, grâce à un bain de saumure, à une température de -10° C. La circulation des gaz est assurée par une pompe péristaltique à travers un circuit constitué de tubes en polytétrafluoroéthylène. A la sortie du réacteur, un réfrigérant enveloppant le tube est alimenté en mélange acétone-carboglace par une pompe, en vue de condenser les vapeurs de fluorure d'hydrogène qui pourraient s'échapper du milieu. En aval de ce tube, un piège à chaux sodée en polytétrafluoroéthylène permet d'arrêter les dernières traces de fluorure d'hydrogène. Après 60 minutes de fonctionnement, on prélève par le tube plongeant dans le réacteur une fraction de la phase liquide en vue de son analyse par résonance magnétique nucléaire du proton. Cette analyse permet d'identifier les cations formés au cours de la réaction. Le spectre de résonance magnétique nucléaire obtenu (signaux à 2,1 ppm et 4,4 ppm) révèle la présence des cations isobutyryle et propionyle et permet d'évaluer leurs proportions respectives, notées sous les rubriques AIB et AP dans le tableau II ci-après. A la phase liquide obtenue après 60 minutes de réaction on ajoute une quantité de fluorure d'hydrogène anhydre telle que le rapport molaire HF/SbF$_5$ devienne égal à 54. A nouveau on prélève une fraction de la phase ainsi obtenue en vue de son analyse par résonance magnétique nucléaire du proton. Le spectre obtenu (signaux à 1,8 ppm et 3,5 ppm) révèle entr'autres la présence de fluorure d'isobutyryle.

5

EP 0 272 945 B1

## TABLEAU II

| Exemple | $CO/C_3H_8$ | AIB | AP |
|---------|-------------|-----|-----|
| 5 | 3,0 | 41 | 59 |
| 6 | 2,0 | 55 | 45 |
| 7 | 1,0 | 89 | 10 |
| 8 | 0,5 | 96 | 2 |
| 9 | 0,1 | 97 | 2 |

EXEMPLE 10

Dans un réacteur en polymonochlorotrifluoroéthylène de volume 3 ml contenant un mélange de fluorure d'hydrogène et de pentafluorure d'antimoine dans un rapport molaire $HF/SbF_5$ égal à 7:3, on plonge un tube en polymonochlorotrifluoroéthylène par lequel on introduit, selon un débit de 210 ml/heure, un mélange de monoxyde de carbone et d'isobutane dans un rapport molaire $CO/C_4H_{10}$ égal à 3. Ce réacteur est maintenu sous pression atmosphérique et, grâce à un bain de saumure, à une température de -10°C. la circulation des gaz est assurée par une pompe péristaltique à travers un circuit constitué de tubes en polytétrafluoroéthylène. A la sortie du réacteur, un réfrigérant enveloppant le tube est alimenté en éthanol par un cryostat, en vue de condenser les vapeurs de fluorure d'hydrogène qui pourraient s'échapper du milieu. En aval de ce tube, un piège à chaux sodée en polytétrafluoroéthylène permet d'arrêter les dernières traces de fluorure d'hydrogène. Après 60 minutes de fonctionnement, on prélève par le tube plongeant dans le réacteur une fraction de la phase liquide en vue de son analyse par résonance magnétique nucléaire du proton. Cette analyse permet :
- d'une part de déterminer le taux de conversion égal à la proportion d'isobutane consommé par rapport à la quantité d'isobutane introduite. Ce taux est ici égal à 46 %.
- d'autre part d'identifier les cations formés au cours de la réaction. Le spectre de résonance magnétique nucléaire obtenu (déjà décrit ci-dessus) révèle notamment la présence des cations pivaloyle, isobutyryle et propionyle et permet d'évaluer leurs proportions respectives :
pivaloyle : 88 %
isobutyryle : 2 %
propionyle : 4 %

Ces proportions sont confirmées par ailleurs par analyse chromatographique en phase gazeuse après piégeage du milieu réactionnel dans un mélange d'éthanol et de bicarbonate de sodium.

6

TABLEAU I

| Exemple | T°C | P | CO/C$_3$H$_8$ | T.C. | AIB | AP | Autres | CH$_4$ | C$_2$H$_6$ | H$_2$ |
|---|---|---|---|---|---|---|---|---|---|---|
| 1 | – 6 | 55$^S$ | 9,5 | 33 | 18 | 76 | 6 | 87 | 10 | 3 |
| 2 | –10 | 6$^d$ | 20,0 | 20 | 5 | 76 | 19 | 93 | 7 | 0 |
| 3 | +30 | 62$^S$ | 16,0 | 94 | 12 | 79 | 9 | 89 | 7 | 4 |
| 4 | –10 | 6$^d$ | 3,3 | 10 | 23 | 66 | 11 | 79 | 21 | 0 |

**Revendications**

1. Procédé catalytique de fabrication de fluorure d'acide à partir de monoxyde de carbone, de fluorure d'hydrogène et d'un flux d'hydrocarbures aliphatiques comprenant à titre principal un alcane comprenant 3 à 4 atomes de carbone, caractérisé par la succession des étapes suivantes :

EP 0 272 945 B1

(a) introduction d'au moins un fluide choisi parmi le monoxyde de carbone et ledit flux d'hydrocarbures dans un réacteur en présence d'un système catalytique superacide constitué essentiellement de fluorure d'hydrogène et de pentafluorure d'antimoine,

(b) le cas échéant, s'il n'a pas déjà été introduit au cours de l'étape (a), introduction dans le réacteur d'un fluide choisi parmi le monoxyde de carbone et ledit flux d'hydrocarbures, le réacteur étant soumis à une température au plus égale à 60°C de manière à former à titre principal un complexe constitué du cation alkyloxocarbonium et de l'anion $SbF_6^-$,

(c) conversion dudit complexe en fluorure d'acide,

(d) séparation du fluorure d'acide, et

(e) récupération du système catalytique superacide.

2. Procédé selon la revendication 1, caractérisé en ce que le rapport molaire CO/alcane dans le réacteur est au moins égal à 0,1.

3. Procédé selon la revendication 2, caractérisé en ce que le rapport molaire CO/alcane dans le réacteur est compris entre 91 et 30.

4. Procédé selon l'une des revendications 1 à 3, caractérisé en ce que le rapport molaire $HF/SbF_5$ dans le réacteur est compris entre 1 et 30.

5. Procédé selon l'une des revendications 1 à 4, caractérisé en ce que la température réactionnelle est comprise entre -80°C et +60°C.

6. Procédé selon l'une des revendications 1 à 5, caractérisé en ce que le complexe formé à l'issue de l'étape (b) est converti en fluorure d'acide au cours de l'étape (c) par intervention d'au moins un moyen pour déplacer l'équilibre entre ledit complexe et le fluorure d'acide vers la formation de ce dernier.

7. Procédé selon la revendication 6, caractérisé en ce que le moyen d'intervention de l'étape (C) est l'addition d'une espèce chimique capable de diminuer significativement l'acidité du milieu réactionnel.

8. Procédé selon la revendication 7, caractérisé en ce que ladite espèce chimique est le fluorure d'hydrogène.

9. Procédé selon l'une des revendications 1 à 8, caractérisé en ce que le système catalytique superacide récupéré au cours de l'étape (e) est recyclé vers le réacteur.

10. Procédé selon les revendications 8 et 9, caractérisé en ce que le recyclage du système catalytique superacide est effectué après élimination partielle de fluorure d'hydrogène.

11. Procédé selon la revendication 10, caractérisé en ce que le fluorure d'hydrogène éliminé à ce stade est recyclé vers le point d'intervention de l'étape (c).

12. Procédé selon l'une des revendications 10 et 11, caractérisé en ce qu'il comprend en outre une étape (f) d'ajustement de la quantité de fluorure d'hydrogène à la constitution du système catalytique superacide mis en oeuvre dans l'étape (a).

## Claims

1. Catalytic process for the manufacture of acid fluoride from carbon monoxide, hydrogen fluoride and an aliphatic hydrocarbon stream comprising principally an alkane containing 3 to 4 carbon atoms, characterised by the following series of stages:

(a) introduction of at least one fluid chosen from carbon monoxide and the said hydrocarbon stream into a reactor in the presence of a superacidic catalyst system consisting essentially of hydrogen fluoride and antimony pentafluoride,

(b) where appropriate, if it has not already been introduced during stage (a), introduction into the reactor of a fluid chosen from carbon monoxide and the said hydrocarbon stream, the reactor being subjected to a temperature of at most 60°C so as to form, principally, a complex consisting of the alkyloxocarbonium cation and the anion $SbF_6^-$,

(c) conversion of the said complex into acid fluoride,
(d) separation of the acid fluoride, and
(e) recovery of the superacidic catalyst system.

2. Process according to Claim 1, characterised in that the molar relationship CO/alkane in the reactor is at least equal to 0.1.

3. Process according to Claim 2, characterised in that the molar relationship CO/alkane in the reactor is between 91 and 30.

4. Process according to one of Claims 1 to 3, characterised in that the molar relationship $HF/SbF_5$ in the reactor is between 1 and 30.

5. Process according to one of Claims 1 to 4, characterised in that the reaction temperature is between $-80\,°C$ and $+60\,°C$.

6. Process according to one of Claims 1 to 5, characterised in that the complex formed at the end of stage (b) is converted into acid fluoride during stage (c) by intervention of at least one means for shifting the equilibrium between the said complex and the acid fluoride towards the formation of the latter.

7. Process according to Claim 6, characterised in that the means of intervention in stage (c) is the addition of a chemical species capable of significantly reducing the acidity of the reaction mixture.

8. Process according to Claim 7, characterised in that the said chemical species is hydrogen fluoride.

9. Process according to one of Claims 1 to 8, characterised in that the superacidic catalyst system recovered during stage (e) is recycled to the reactor.

10. Process according to Claims 8 and 9, characterised in that the recycling of the superacidic catalyst system is performed after partial removal of hydrogen fluoride.

11. Process according to Claim 10, characterised in that the hydrogen fluoride removed at this stage is recycled to the point of intervention in stage (c).

12. Process according to either of Claims 10 and 11, characterised in that it additionally comprises a stage (f) of adjustment of the quantity of hydrogen fluoride to the constitution of the superacidic catalyst system employed in stage (a).

**Patentansprüche**

1. Katalytisches verfahren zur Herstellung von Säurefluorid aus Kohlenmonoxid, Fluorwasserstoff und einem Strom aliphatischer Kohlenwasserstoffe, der als Hauptbestandteil ein Alkan mit 3 bis 4 Kohlenstoffatomen enthält, gekennzeichnet durch die Aufeinanderfolge nachstehender Schritte:
(a) Einbringen mindestens eines Fluids, ausgewählt aus der Gruppe bestehend aus Kohlenmonoxid und dem genannten Strom aus Kohlenwasserstoffen, in einen Reaktor in Gegenwart eines übersauren katalytischen Systems, hauptsächlich bestehend aus Fluorwasserstoff und Antimonpentafluorid,
(b) gegebenenfalls, sofern nicht schon im Laufe des Schrittes (a) eingebracht, Einbringen eines Fluids, ausgewählt aus der Gruppe bestehend aus Kohlenmonoxid und dem genannten Strom aus Kohlenwasserstoffen, in den Reaktor, wobei der Reaktor einer Temperatur von höchstens $60\,°C$ ausgesetzt wird, um als Hauptbestandteil einen aus dem Alkyloxocarbonium-Kation und dem $SbF_6^-$-Anion bestehenden Komplex zu bilden,
(c) Umwandeln des genannten Komplexes zu Säurefluorid,
(d) Abtrennen des Säurefluorids, und
(e) Rückgewinnen des übersauren katalytischen Systems.

2. Verfahren nach Anspruch 1, dadurch gekennzeichnet, daß das Molverhältnis CO/Alkan in dem Reaktor mindestens 0,1 beträgt.

3. Verfahren nach Anspruch 2, dadurch gekennzeichnet, daß das Molverhältnis CO/Alkan in dem Reaktor zwischen 91 und 30 beträgt.

4. Verfahren nach einem der Ansprüche 1 bis 3, dadurch gekennzeichnet, daß das Molverhältnis HF/SbF$_5$ in dem Reaktor zwischen 1 und 30 beträgt.

5. Verfahren nach einem der Ansprüche 1 bis 4, dadurch gekennzeichnet, daß die Reaktionstemperatur zwischen -80°C und +60°C beträgt.

6. Verfahren nach einem der Ansprüche 1 bis 5, dadurch gekennzeichnet, daß der am Ende des Schrittes (b) gebildete Komplex während des Schrittes (c) durch Intervention mindestens eines Mittels zum Verschieben des Gleichgewichts zwischen dem genannten Komplex und dem Säurefluorid in Richtung der Bildung des letzteren zu Säurefluorid umgewandelt wird.

7. Verfahren nach Anspruch 6, dadurch gekennzeichnet, daß das Interventionsmittel des Schrittes (c) die Zugabe einer chemischen Spezies ist, die imstande ist, den Säuregehalt des Reaktionsmilieus bedeutend zu verringern.

8. Verfahren nach Anspruch 7, dadurch gekennzeichnet, daß die genannte chemische Spezies Fluorwasserstoff ist.

9. Verfahren nach einem der Ansprüche 1 bis 8, dadurch gekennzeichnet, daß das im Schritt (e) rückgewonnene übersaure katalytische System zum Reaktor rückgeführt wird.

10. Verfahren nach den Ansprüchen 8 und 9, dadurch gekennzeichnet, daß die Rückführung des übersauren katalytischen Systems nach der teilweisen Eliminierung des Fluorwasserstoffs erfolgt.

11. Verfahren nach Anspruch 10 oder 11, dadurch gekennzeichnet, daß der in diesem Stadium eliminierte Fluorwasserstoff zum Interventionspunkt des Schrittes (c) rückgeführt wird.

12. Verfahren nach Anspruch 10, dadurch gekennzeichnet, daß es weiters einen Schritt (f) zum Anpassen der Menge an Fluorwasserstoff an die Zusammensetzung des im Schritt (a) eingesetzten übersauren katalytischen Systems umfaßt.